# EUROPÄISCHE PATENTSCHRIFT

(11) **EP 0 642 802 B1**
(45) Veröffentlichungstag und Bekanntmachung des Hinweises auf die Patenterteilung: **28.06.2000**
(21) Anmeldenummer: 94112250.9
(22) Anmeldetag: 05.08.1994
(51) Int. Cl.: A61M 15/00, B05B 17/06

(54) **Inhalationsgerät**
Inhalation device
Dispositif d'inhalation

(30) Priorität: 09.08.1993 CH 236993
(43) Veröffentlichungstag der Anmeldung: 15.03.1995
(73) Patentinhaber: SIEMENS AKTIENGESELLSCHAFT, 80333 München (DE)
(72) Erfinder: Van der Linden, Klaus, Dipl.-Ing., D-96257 Redwitz (DE); Rüttel, Martin, Dipl.-Ing. (FH), D-96271 Grub (DE); Haack, Olaf, Dipl.-Phys., D-96231 Staffelstein (DE)

(56) Entgegenhaltungen:
- EP-A- 0 004 039
- EP-A- 0 373 237
- WO-A-93/12823
- DE-A- 3 841 442
- GB-A- 2 099 710
- US-A- 3 812 854

## Beschreibung

Die Erfindung bezieht sich auf ein medizinisches Inhalationsgerät zur Erzeugung einer über die Atmungswege aufzunehmenden Aerodispersion von pharmazeutisch wirksamen Substanzen, gemäss dem Oberbegriff des Patentanspruchs 1.

Ein solches Gerät ist bereits aus der EP-B1 0 258 637 bekannt. Der Dosiervorgang erfolgt bei diesem bekannten Gerät durch manuelle Betätigung eines Druckknopfes mit dem die das Medikament enthaltende Flüssigkeitskartusche etwas zusammengedrückt wird und dadurch eine gewisse Menge Medikament in Tropfenform an den Zerstäuber abgibt. Bei einem solchen handbetätigten Dosiervorgang variiert der erzeugte Druck auf die Kartusche innerhalb weiter Grenzen, so dass die ausgeschüttete Medikamentenmenge nicht vorausbestimmbar ist. Wird die Austrittsgeschwindigkeit der Medikamentenflüssigkeit zu gross, so entsteht statt der gewünschten Tropfenform ein kontinuierlicher Flüssigkeitsstrahl, so dass die Medikamentenflüssigkeit nicht mehr auf den Teller des Zerstäubers absetzbar ist. Ein weiterer Nachteil dieses bekannten Gerätes besteht in der Boschränkung der Dosiermöglichkeit auf einen einzigen Tropfen, d.h. auf ca. 20 µl.

Weitere Inhalationsgeräte auf Ultraschallbasis sind aus der US 3,812,854, der GB 2 099 710 A und der DE 38 41 442 C2 bekannt.

Der Erfindung liegt die Aufgabe zugrunde, ein Inhalationsgerät zu schaffen, welches durch eine kontrollierte und mit dem Ultraschallzerstäuber koordinierte Ausschüttung von Medikamentenflüssigkeit eine optimale Zerstäubung des Medikaments gestattet.

Weitere Aufgaben bestanden in der Verhinderung einer Verdunstung oder Kristallisation der Medikamentenflüssigkeit auf dem Zuführweg zum Zerstäuber; eine Verbesserung der Lagerfähigkeit der Medikamentenflüssigkeit und die Modularisierung des Inhalationsgerätes, um verschiedene Medikamente mit dem gleichen Gerät applizieren zu können.

Die Erfindung löst die gestellte Aufgabe mit einem Inhalationsgerät, welches die Merkmale des Anspruchs 1 aufweist.

Weitere vorteilhafte Ausgestaltungen der Erfindung sind in den abhängigen Ansprüchen gekennzeichnet.

Durch entsprechende Steuerung der Fördervorrichtung können
a) Einzeltropfen
b) eine Serie von aufeinanderfolgenden diskreten Einzeltropfen und
c) ein kontinuierlicher, feiner Flüssigkeitsstrahl (mit definierter Flüssigkeitsmenge pro Zeiteinheit) an den Ultraschallzerstäuber abgegeben werden.

Bevorzugt werden "Tropfeneinheiten" von 10 - 20 µl auf den Teller des Zerstäubers getropft. Vorzugsweise werden auf diese Weise innerhalb von 1,5 Sekunden 50 µl gefördert und zerstäubt. Je nach Medikamentenart können aber Tropfen von 3 - 40 µl vorteilhaft sein.

Die Erfindung und Weiterbildungen der Erfindung werden im folgenden anhand der teilweise schematischen Darstellungen mehrerer Ausführungsbeispiele noch näher erläutert.

Es zeigen:
- Fig. 1: einen Querschnitt durch ein erfindungsgemässes Inhalationsgerät mit eingelegter aktivierter Dosiereinheit nach Fig. 3;
- Fig. 2: einen Querschnitt durch eine Dosiereinheit für das erfindungsgemässe Inhalationsgerät im inaktiven Lagerzustand;
- Fig. 3: einen Querschnitt durch eine Dosiereinheit für das erfindungsgemässe Inhalationsgerät im aktivierten Zustand;
- Fig. 4: einen Detailquerschnitt durch die Transferleitung des Inhalationsgerätes nach Fig. 3.

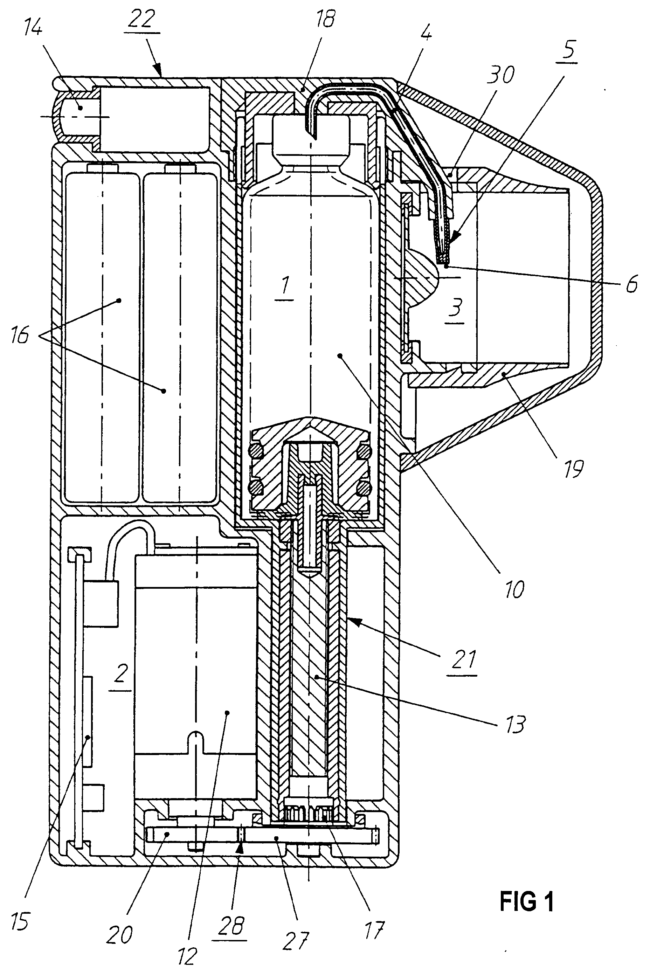

Das in Fig. 1 dargestellte medizinische Inhalationsgerät besteht aus einer separaten Dosiereinheit 21, welche in ein die Fördervorrichtung 2, den Ultraschallzerstäuber 3 und die Ablaufsteuerung 15 enthaltendes Gehäuse 22 einsetzbar und nach Belieben wieder entfernbar ist.

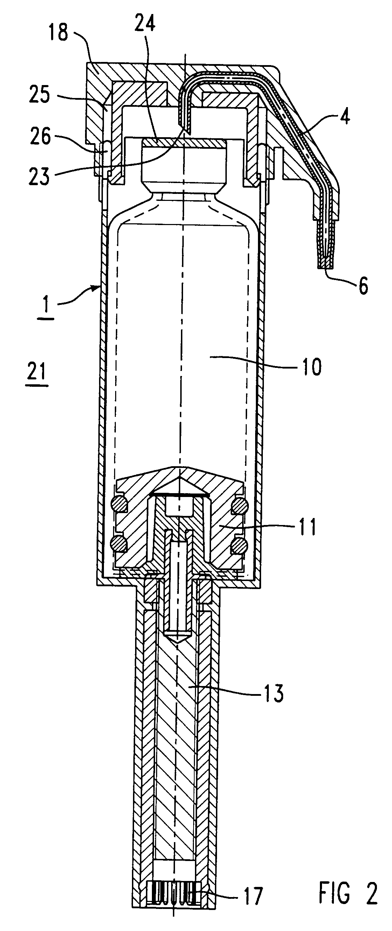

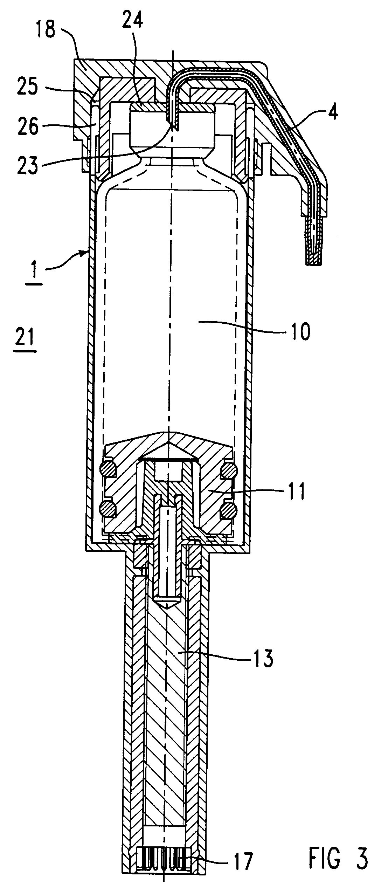

Die in den Fig. 2 und 3 dargestellte Dosiereinheit 21 umfasst einen Vorratsbehälter 1 zur Aufnahme einer die Medikamentenflüssigkeit enthaltenden Spritzampulle 10 mit beweglichem Kolben 11, in welchen die Gewindestange 13 eingreift. Der Vorratsbehälter 1 ist im ringförmigen Hohlraum 25 eines die Transferleitung 4 tragenden Adapterkopfes 18 mit seiner ebenfalls ringförmigen Sperrklinke 26 eingesetzt.

In der in Fig. 2 dargestellten inaktivierten Position ist die Spritzampulle 10 noch völlig intakt, so dass ihre Lagerfähigkeit unbeeinträchtigt ist. Wird der die Spritzampulle 10 enthaltende Vorratsbehälter 1 durch eine rotatorische oder translatorische Bewegung in den ringförmigen Hohlraum 25 des Adapterkopfes 18 hineingedrückt, so wird dabei die Ampullenmembrane 24 von dem dem Vorratsbehälter 1 zugewandten Ende 23 der Transferleitung 4 durchbohrt, so dass Medikamentenflüssigkeit von der Spritzampulle 10 in die Transferleitung 4 einfliessen kann.

Die derart aktivierte Dosiereinheit 21 (Fig. 3) kann nun verdrehungssicher in das Gehäuse 22 (Fig. 1) eingelegt werden, wobei ein am freien Ende der Gewindestange 13 angebrachter Konnektor 17 mit einem Zahnrad 27 des Getriebes 28 der Fördervorrichtung 2 verbunden wird (das Zahnrad 27 steht seinerseits im Eingriff mit dem Zahnrad 20 des Getriebes 28, welches vom Antriebsaggregat 12, vorzugsweise einem Elektromotor, angetrieben wird). Das andere freie Ende 6 der Transferleitung 4 wird dabei durch eine Öffnung 30 im Mundstück 19 auf eine genau vordefinierte Position unmittelbar über dem Ultraschallzerstäuber 3 positioniert.

Der Ultraschallzerstäuber 3 umfasst ein piezoelektrisches Schwingsystem, welches Ultraschall-Biegeschwingungen erzeugt. Ein geeignetes Ultraschall-Schwingsystem ist in der EP-B1 246.515 beschrieben.

Die Fördervorrichtung 2 umfasst, wie in Fig. 1 dargestellt, das Antriebsaggregat 12 mit dem Getriebe 28. Die beiden Batterie-Akkus 16 speisen sowohl das Antriebsaggregat 12 als auch die elektronische Ablaufsteuerung 15. Die Steuerung des Antriebsaggregates 12 und des Ultraschallzerstäubers 3 erfolgt durch die Ablaufsteuerung 15, vorzugsweise einem programmierbaren Mikroprozessor, durch Drücken des Betätigungsknopfes 14 (statt des Betätigungsknopfes 14 kann bei anspruchsvolleren Anwendungen ein Tastaturfeld verwendet werden).

Ein für das erfindungsgemässe Inhalationsgerät geeignetes Dosiersystem als Teil der Fördervorrichtung ist in der EP-B1 143.895 beschrieben und besteht aus einem Spindel/Mutter-Getriebe, wobei das Antriebsglied des Getriebes durch eine im Gerätegehäuse drehbar gelagerte Mitnehmerhülse und das Abtriebsglied durch die Spindel gebildet ist. Die Mitnehmerhülse ist koaxial zu einer am Gerätegehäuse vorgesehenen Halterung für die Spritzampulle angeordnet und die Spindel ist koaxial in die Mitnehmerhülse einführbar. Die Mutter ist an einem an die Halterung angrenzenden Teil des Gehäuses gegen axiale Verschiebung abstützbar und drehfest halterbar. Die Spindel ist in der Mitnehmerhülse längsbeweglich und drehfest damit die Spindel für den unmittelbaren Vorschub des Kolbens einer Spritzampulle durch Drehen der Mitnehmerhülse in die Halterung vorschiebbar ist.

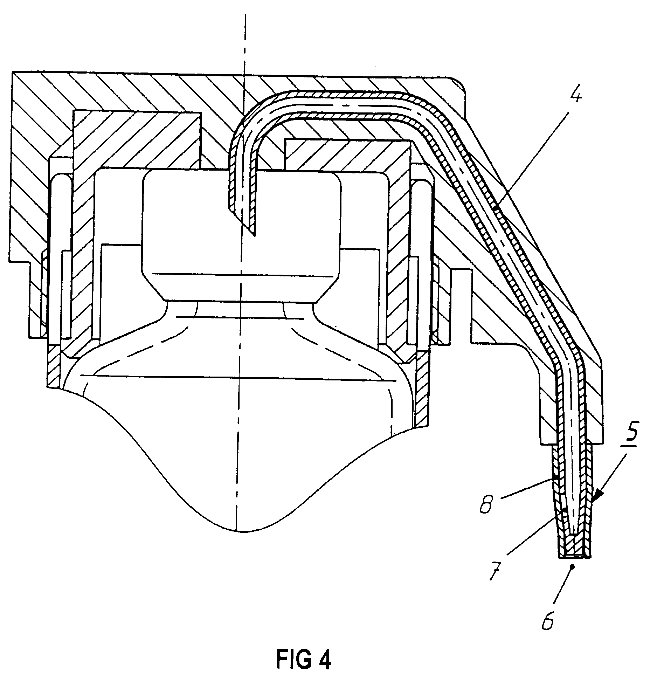

Die unmittelbar über dem Ultraschallzerstäuber 3 endende Transferleitung 4 ist - wie in Fig. 4 gezeigt - im Ruhezustand luftdicht verschlossen. Das dem Ultraschallzerstäuber 3 zugewandte Ende 6 der Transferleitung 4 ist geschlossen und weist lediglich eine seitlich im Mantel der Transferleitung 4 angebrachte Öffnung 7 auf, welche durch eine elastische Membrane 8 in Form eines über das Ende 6 der Transferleitung 4 gestülpten Gummischlauches, vorzugsweise aus Silikongummi, verschlossen ist. Diese Konstruktion wirkt als ein im Ruhezustand selbsttätig geschlossenes Ventil 5, welches sich im Betriebszustand durch den Flüssigkeitsdruck selbsttätig öffnet. Durch Aktivierung der Fördervorrichtung 2 wird aus dem Vorratsbehälter 1 Medikamentenflüssigkeit durch die Transferleitung 4 gefördert und auf den Ultraschallzerstäuber 3 transferiert. Die Fördervorrichtung 2 ist derart ausgelegt, dass sie einen Arbeitsdruck von 0,01 - 5,00 bar, vorzugsweise von 0,5 - 1,0 bar, in der Spritzampulle 10 erzeugt.

Die Applikation des Inhalationsgeräts durch den Patienten erfolgt derart, dass das Inhalationsgerät mit seinem Mundstück 19 an den Mund gebraucht wird. Wird nun der Betätigungsknopf 14 gedrückt, so aktiviert dieser über die Ablaufsteuerung 15 sowohl die Fördervorrichtung 2 (mit dem Antriebsaggregat 12 und dem Getriebe 28) als auch den Ultraschallzerstäuber 3. Die Aktivierung des Ultraschallzerstäubers 3 kann entweder simultan mit der Fördervorrichtung 2 erfolgen oder mit einer positiven oder negativen Verzögerung. Das Getriebe 28 betätigt über die beiden Zahnräder 20, 27 den Konnektor 17 die Gewindestange 13, welche nach oben verschoben wird, so dass der Kolben 11 der Spritzampulle 10 in Axialrichtung nach oben gedrückt wird und Medikamentenflüssigkeit in die Transferleitung 4 eingespeist wird. Die über das Ende 6 der Transferleitung 4 gestülpte, elastische Membrane 8 wird an der Öffnung 7 radial aufgeweitet, so dass Medikamentenflüssigkeit austreten und sich am Ende 6 der Transferleitung 4 zu einem Tropfen ausbilden kann, der durch die hochfrequenten Schwingungen des Ultraschallzerstäubers 3 zerstäubt und durch das Mundstück 129 freigesetzt wird. Durch Aktivierung des Betätigungsknopfes 14 und gleichzeitiges Einatmen am Mundstück 19 gelangt somit das nun lungengängige Medikament in kontrollierter Menge in die Atemwege des Patienten.

Bei Verwendung eines Mikroprozessors als Ablaufsteuerung 15 kann der Betätigungsknopf 14 in Form einer Bedienungstastatur mit mehreren Funktionen und gegebenenfalls einer Programmierung ausgebildet werden. Damit ist es möglich, mehrere beliebig definierbare Mengen an zerstäubtem Medikament auszuschütten.

Andererseits ist es auch möglich, durch den Einsatz verschiedenartiger Dosiereinheiten 21 beliebige Arten und Mengen von Medikamenten mit dem gleichen Gehäuse 22 zu applizieren, wobei die einzelnen Dosiereinheiten 21 als Einweg-Systeme konzipiert sind.

## Patentansprüche

1. Medizinisches Inhalationsgerät zur Erzeugung einer über die Atmungswege aufzunehmenden Aerodispersion von pharmazeutisch wirksamen Substanzen, mit einem Vorratsbehälter (1) für die als Flüssigkeit vorliegende pharmazeutisch wirksame Substanz, einer Fördervorrichtung (2) für den Ausstoss der Flüssigkeit aus dem Vorratsbehälter (1), einem Ultraschallzerstäuber (3) und einer zwischen dem Vorratsbehälter (1) und dem Ultraschallzerstäuber (3) angeordneten Transferleitung (4) für die Flüssigkeit, wobei das Inhalationsgerät über eine Ablaufsteuerung (15) verfügt, mit welcher die Fördervorrichtung (2) und der Ultraschallzerstäuber (3) steuerbar ist, **dadurch gekennzeichnet,** dass die Transferleitung (4) an ihrem dem Ultraschallzerstäuber (3) zugewandten Ende (6) eine im Mantel der Transferleitung (4) angebrachte Öffnung (7) aufweist, welche durch eine elastische Membrane (8) verschlossen ist.

2. Inhalationsgerät nach Anspruch 1,
**dadurch gekennzeichnet,** dass die Ablaufsteuerung (15) ein Mikroprozessor ist.

3. Inhalationsgerät nach Anspruch 1 oder 2,
**dadurch gekennzeichnet,** dass die Ablaufsteuerung (15) programmierbar ist.

4. Inhalationsgerät nach einem der Ansprüche 1 bis 3,
**dadurch gekennzeichnet,** dass die Transferleitung (4) die Öffnung (7) seitlich aufweist, welche in Form eines über das Ende (6) der Transferleitung (4) gestülpten Gummischlauches verschlossen ist.

5. Inhalationsgerät nach einem der Ansprüche 1 bis 4,
**dadurch gekennzeichnet,** dass der Ultraschallzerstäuber (3), vorzugsweise mittels eines piezoelektrischen Schwingsystems, Ultraschall-Biegeschwingungen erzeugt.

6. Inhalationsgerät nach einem der Ansprüche 1 bis 5,
**dadurch gekennzeichnet,** dass der Vorratsbehälter (1) eine Spritzampulle (10) mit beweglichem Kolben (11) umfasst.

7. Inhalationsgerät nach Anspruch 6,
**dadurch gekennzeichnet,** dass eine Gewindestange (13) vorgesehen ist, die in den Kolben (11) eingreift, und die an ihrem dem Kolben (11) abgewandten Ende einen Konnektor (17) zur Verbindung mit einem Getriebe (28) aufweist.

8. Inhalationsgerät nach Anspruch 7,
**dadurch gekennzeichnet,** dass der Vorratsbehälter (1) mit Spritzampulle (10), die Transferleitung (4) sowie die den Konnektor (17) enthaltende Gewindestange (13) zusammen als eine separate Dosiereinheit (21) ausgebildet sind, welche in ein die Fördervorrichtung (2), den Ultraschallzerstäuber (3) und die Ablaufsteuerung (15) enthaltendes Gehäuse (22) einsetz- und entfernbar ist.

9. Inhalationsgerät nach einem der Ansprüche 6 bis 8,
**dadurch gekennzeichnet,** dass der die Spritzampulle (10) enthaltende Vorratsbehälter (1) mit einem die Transferleitung (4) umfassenden Adapterkopf (18) versehen ist, wobei der Adapterkopf (18) derart mit dem Vorratsbehälter (1) kuppelbar ist, dass das dem Vorratsbehälter (1) zugewandten Ende (23) der Transferleitung (4) eine Ampullenmembrane (24) der Spritzampulle (10) durchsticht und Medikamentenflüssigkeit von der Spritzampulle (10) in die Transferleitung (4) einfliessen kann.

10. Inhalationsgerät nach einem der Ansprüche 1 bis 9,
**dadurch gekennzeichnet,** dass die Fördervorrichtung (2) und der Ultraschallzerstäuber (3) simultan aktivierbar sind.

11. Inhalationsgerät nach einem der Ansprüche 1 bis 9,
**dadurch gekennzeichnet,** dass die Fördervorrichtung (2) gegenüber dem Ultraschallzerstäuber (3) mit zeitlicher Verzögerung aktivierbar ist.

12. Inhalationsgerät nach einem der Ansprüche 1 bis 9,
**dadurch gekennzeichnet,** dass der Ultraschallzerstäuber (3) gegenüber der Fördervorrichtung (2) mit zeitlicher Verzögerung aktivierbar ist.

## Claims

1. Medical inhalation apparatus for generating an aero-dispersion of pharmaceutically active substances to be taken in via the respiratory tract, having a storage container (1) for the pharmaceutically active substance, which is present in liquid form, a conveyor device (2) for expelling the liquid from the storage container (1), an ultrasonic atomiser (3) and a transfer line (4) for the liquid that is arranged between the storage container (1) and the ultrasonic atomiser (3), the inhalation apparatus having a sequence control (15) with which the conveyor device (2) and the ultrasonic atomiser (3) can be controlled, characterised in that the transfer line (4) has at its end (6) which faces the ultrasonic atomiser (3) an opening (7) made in the casing of the transfer line (4), which opening is closed by an elastic membrane (8).

2. Inhalation apparatus according to claim 1, characterised in that the sequence control (15) is a microprocessor.

3. Inhalation apparatus according to claim 1 or 2, characterised in that the sequence control (15) is programmable.

4. Inhalation apparatus according to one of claims 1 to 3, characterised in that the transfer line (4) has the opening (7) at the side, which opening is closed in the form of a rubber tube pulled over the end (6) of the transfer line (4).

5. Inhalation apparatus according to one of claims 1 to 4, characterised in that the ultrasonic atomiser (3), preferably by means of a piezoelectric vibration system, generates ultrasonic bending vibrations.

6. Inhalation apparatus according to one of claims 1 to 5, characterised in that the storage container (1) comprises a spray ampoule (10) having a movable piston (11).

7. Inhalation apparatus according to claim 6, characterised in that there is provided a threaded rod (13) which engages in the piston (11) and has at the end facing away from the piston (11) a connector (17) for connection to a gearing (28).

8. Inhalation apparatus according to claim 7, characterised in that the storage container (1) with spray ampoule (10), the transfer line (4) and the threaded rod (13) containing the connector (17) are together constructed as a separate dosing unit (21), which can be put into and removed from a housing (22) containing the conveyor device (2), the ultrasonic atomiser (3) and the sequence control (15).

9. Inhalation apparatus according to one of claims 6 to 8, characterised in that the storage container (1) containing the spray ampoule (10) is provided with an adapter head (18) which surrounds the transfer line (4), wherein the adapter head (18) can be coupled to the storage container (1) in such a way that the end (23) of the transfer line (4) that faces the storage container (1) penetrates an ampoule membrane (24) of the spray ampoule (10) and medicament liquid can flow from the spray ampoule (10) into the transfer line (4).

10. Inhalation apparatus according to one of claims 1 to 9, characterised in that the conveyor device (2) and the ultrasonic atomiser (3) can be activated simultaneously.

11. Inhalation apparatus according to one of claims 1 to 9, characterised in that the conveyor device (2) can be activated with a time delay with respect to the ultrasonic atomiser (3).

12. Inhalation apparatus according to one of claims 1 to 9, characterised in that the ultrasonic atomiser (3) can be activated with a time delay with respect to the conveyor device (2).

## Revendications

1. Dispositif médical d'inhalation pour produire une aérodispersion de substances actives du point de vue pharmaceutique s'absorbant par l'intermédiaire des voies respiratoires, comportant un récipient (1) de réserve pour la substance active du point de vue pharmaceutique se présentant sous la forme d'un liquide, un dispositif (2) de transport pour l'expulsion du liquide hors du récipient (1) de réserve, un atomiseur (3) à ultrasons et une conduite (4) de transport pour le liquide montée entre le récipient (1) de réserve et l'atomiseur (3) à ultrasons, le dispositif d'inhalation disposant d'une commande (15) d'écoulement par laquelle le dispositif (2) de transport et l'atomiseur (3) à ultrasons peuvent être commandés, caractérisé en ce que la conduite (4) de transport comporte, à son extrémité (6) tournée vers l'atomiseur (3) à ultrasons, une ouverture qui est ménagée dans la surface latérale de la conduite (4) de transport et qui est fermée par une membrane (8) élastique.

2. Dispositif d'inhalation suivant la revendication 1, caractérisé en ce que la commande (15) d'écoulement est un microprocesseur.

3. Dispositif d'inhalation suivant la revendication 1 ou 2, caractérisé en ce que la commande (15) d'écoulement est programmable.

4. Dispositif d'inhalation suivant l'une des revendications 1 à 3, caractérisé en ce que la conduite (4) de transport comporte latéralement l'ouverture (7) dont la fermeture est en forme d'un tuyau souple en caoutchouc retroussé sur l'extrémité (6) de la conduite (4) de transport.

5. Dispositif d'inhalation suivant l'une des revendications 1 à 4, caractérisé en ce que l'atomiseur (3) à ultrasons produit des vibrations de flexion à ultrasons, de préférence au moyen d'un système vibratoire piézoélectrique.

6. Dispositif d'inhalation suivant l'une des revendications 1 à 5, caractérisé en ce que le récipient (1) de réserve comporte une ampoule (10) de pulvérisation à piston (11) mobile.

7. Dispositif d'inhalation suivant la revendication 6, caractérisé en ce qu'il est prévu une tige (13) filetée qui rentre dans le piston (11) et qui comporte à son extrémité éloignée du piston (11) un connecteur (17) pour la liaison à une transmission (28).

8. Dispositif d'inhalation suivant la revendication 7, caractérisé en ce que le récipient (1) de réserve à ampoule (10) de pulvérisation, la conduite (4) de transport ainsi que la tige (13) filetée comportant le connecteur (17) sont réalisés conjointement en une unité (21) de dosage distincte qui peut être introduite dans un boîtier (22), comportant le dispositif (2) de transport, l'atomiseur (3) à ultrasons et la commande (15) d'écoulement, et en être retirée.

9. Dispositif d'inhalation suivant l'une des revendications 6 à 8, caractérisé en ce que le récipient (1) de réserve contenant l'ampoule (10) de pulvérisation est muni d'une tête (18) d'adaptateur comprenant la conduite (4) de transport, la tête (18) d'adaptateur pouvant être coupée au récipient (1) de réserve de telle manière que l'extrémité (23) de la conduite (4) de transport tournée vers le récipient (1) de réserve perce une membrane (24) de l'ampoule (10) de pulvérisation et que du liquide médicamenteux peut s'écouler de l'ampoule (10) de pulvérisation à la conduite (4) de transport.

10. Dispositif d'inhalation suivant l'une des revendications 1 à 9, caractérisé en ce que le dispositif (2) de transport et l'atomiseur (3) à ultrasons peuvent être activés en même temps.

11. Dispositif d'inhalation suivant l'une des revendications 1 à 9, caractérisé en ce que le dispositif (2) de transport peut être activé avec retard dans le temps par rapport à l'atomiseur (3) à ultrasons.

12. Dispositif d'inhalation suivant l'une des revendications 1 à 9, caractérisé en ce que l'atomiseur (3) à ultrasons peut être activé avec un retard dans le temps par rapport au dispositif (2) de transport.
